(19) Europäisches Patentamt
European Patent Office
Office européen des brevets

(11) **EP 2 635 719 B1**

(12) **FASCICULE DE BREVET EUROPEEN**

(45) Date de publication et mention de la délivrance du brevet:
**04.04.2018 Bulletin 2018/14**

(51) Int Cl.:
***C22C 14/00*** *(2006.01)*  ***A61C 8/00*** *(2006.01)*
***C22F 1/18*** *(2006.01)*

(21) Numéro de dépôt: **11799830.2**

(22) Date de dépôt: **04.11.2011**

(86) Numéro de dépôt international:
**PCT/IB2011/054939**

(87) Numéro de publication internationale:
**WO 2012/059895 (10.05.2012 Gazette 2012/19)**

(54) **MATERIAU METALLIQUE A GRADIENT D'ELASTICITE**

**METALLISCHER WERKSTOFF MIT EINEM ELASTIZITÄTSGRADIENTEN**

**A METALLIC MATERIAL HAVING A GRADED ELASTICITY**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.11.2010 FR 1004327**

(43) Date de publication de la demande:
**11.09.2013 Bulletin 2013/37**

(73) Titulaire: **Centre National de la Recherche Scientifique**
**75016 Paris (FR)**

(72) Inventeurs:
• **PRIMA, Frédéric**
**F-75014 Paris (FR)**
• **NOWAK, Sophie**
**F-75014 Paris (FR)**

(74) Mandataire: **Gevers & Orès**
**41 avenue de Friedland**
**75008 Paris (FR)**

(56) Documents cités:
**US-A- 5 447 580     US-A1- 2007 137 742**

• **SUN F ET AL: "Influence of a short thermal treatment on the superelastic properties of a titanium-based alloy", SCRIPTA MATERIALIA, ELSEVIER, AMSTERDAM, NL, vol. 63, no. 11, 1 novembre 2010 (2010-11-01), pages 1053-1056, XP027289858, ISSN: 1359-6462 [extrait le 2010-08-02]**

EP 2 635 719 B1

Description

La présente invention concerne un matériau métallique à gradient d'élasticité, son procédé de préparation et son utilisation, notamment pour la réalisation d'implants dentaires.

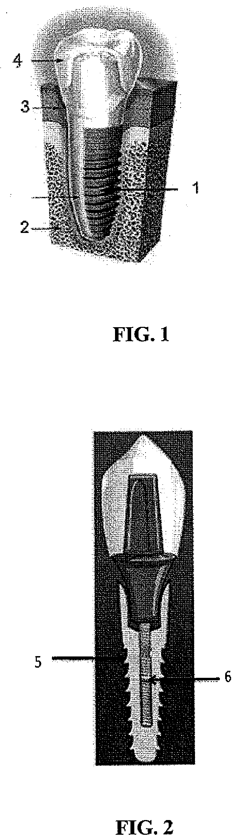

Les implants dentaires couramment utilisés se composent généralement d'un corps d'implant, fileté extérieurement, destiné à être mis en place par vissage dans l'os mandibulaire ou maxillaire, et d'une pièce rapportée sur le corps, dite faux-moignon ou pilier, destinée à être fixée sur le corps de l'implant, après l'implantation, et aménagée pour recevoir une prothèse dentaire (Cf. figures 1 et 2).

Les implants dentaires sont généralement constitués d'un alliage de titane (à titre d'exemple: Ti-6Al-4V) dont le module d'élasticité (« module d'Young ») se situe aux alentours de 90-110 GPa. Toutefois, l'écart avec le module d'élasticité de l'os humain (30 GPa) est important, de sorte que les contraintes résultant de la mastication ne sont pas transférées de manière satisfaisante à l'os entourant l'implant, ce qui peut provoquer au final une désorption de l'os et la perte de l'implant dans certains cas cliniques non favorables.

De fait, ce mauvais transfert de contrainte peut induire soit une stimulation mécanique insuffisante, soit au contraire des pics de contraintes et de déformations au niveau de l'os péri-implantaire. Dans un cas comme dans l'autre, cela peut nuire à l'ostéointégration de l'implant et constitue une limitation à l'utilisation des implants dans certaines parties de la sphère buccale, notamment dans les sites où l'os est en faible quantité ou de qualité insuffisante.

Il a maintenant été mis au point un matériau possédant un gradient d'élasticité. Plus précisément, ce matériau se caractérise par des propriétés superélastiques en surface et élastiques à coeur, le module d'élasticité apparent en surface étant par ailleurs beaucoup plus faible que celui au coeur du matériau.

Ce matériau est particulièrement utile pour la réalisation d'un nouveau type d'implants dentaires. Ainsi, il est possible d'obtenir un matériau selon l'invention possédant sur la face externe de l'implant (interface implant/os) un module apparent d'environ 30 GPa très proche de celui de l'os, et sur la face interne de ce même implant (interface implant/pilier) un module d'environ 80 GPa permettant d'assurer la rigidité de l'ensemble implant/pilier/prothèse. En assurant un meilleur transfert des contraintes de l'implant à l'os, ce matériau permet de dissiper une partie de l'énergie mécanique dérivant des contraintes fonctionnelles (mastication) et d'éviter les pics de contraintes et de déformation dans l'os péri-implantaire. Il en résulte avantageusement une stimulation du remodelage osseux, et donc une meilleure ostéointégration de l'implant.

De par ces propriétés, ce matériau offre de façon avantageuse, la possibilité de concevoir des implants dentaires pour des sites de la sphère buccale où l'implantation directe n'est généralement pas possible et nécessite de recourir à la chirurgie, par exemple dans le cas des sites implantaires où l'os est en faible quantité ou de qualité insuffisante.

En outre, ce matériau peut être préparé selon des procédés économiques, fiables, faciles à mettre en oeuvre, et aisément transposables à l'échelle industrielle.

L'objet de la présente invention est défini par les revendications 1 à 17.

*Matériau métallique (M) à gradient d'élasticité*

Ainsi, un premier objet de la description concerne un matériau métallique monolithique (M) comprenant, dans un domaine de température ($\Delta T$) et à pression atmosphérique :

- au moins une zone périphérique externe constituée d'une microstructure ($m_1$) ayant un module d'élasticité ($E_1$) et possédant des propriétés superélastiques dans ledit domaine ($\Delta T$), et
- un coeur constitué d'une microstructure ($m_2$) ayant un module d'élasticité ($E_2$), et possédant des propriétés élastiques dans ledit domaine ($\Delta T$) ;
  lesdites microstructures ($m_1$) et ($m_2$) étant distinctes l'une de l'autre, et
  ledit module d'élasticité ($E_1$) étant inférieur audit module d'élasticité ($E_2$).

Tel qu'on l'utilise ici, matériau métallique « monolithique » signifie un matériau constitué d'un seul et même élément métallique ou d'une seule et même combinaison d'éléments métalliques, par exemple un alliage de titane et de niobium (Ti-Nb), à la différence notamment des matériaux dits « composites » qui se caractérisent par la présence d'au moins une interface entre deux matériaux distincts. Cela n'exclut pas que la composition chimique d'un alliage monolithique puisse varier dans les différentes phases cristallines ou mêmes dans les différentes zones de l'échantillon. Le matériau M est notamment une pièce de matériau M, tel qu'un lingot ou un barreau.

Les termes « microstructure » ou « état microstructural » sont employés ici de façon interchangeable, et se réfèrent à la structure interne du matériau métallique, à une échelle généralement comprise entre 10 nm et 100 $\mu$m. La microstructure d'un matériau joue un rôle important puisqu'elle conditionne un grand nombre de propriétés, notamment les propriétés mécaniques du matériau. En règle générale, la microstructure d'un matériau se caractérise principalement par les caractéristiques de la solution solide du matériau, l'arrangement des phases, la taille des grains et la porosité. La solution solide est caractérisée notamment par le caractère cristallin ou amorphe, ordonné ou non de la solution solide ainsi que les défauts, tels que la concentration en lacunes, et les solutés éventuellement présents. Les techniques de caractérisation de la microstructure d'un matériau sont très variées. On distingue notamment les techniques de caractérisation morphologique, et les techniques de caractérisation de la structure des matériaux. Parmi les techniques de caractérisation morphologique, on peut citer notamment la microscopie optique (OM), microscopie électronique à balayage (MEB), ou la microscopie électronique en transmission (MET). Pour plus d'information concernant ces techniques, on pourra se référer notamment à la fiche M260 des Techniques de l'Ingénieur. L'identification des phases se fait généralement par diffraction des rayons X.

Le « module d'élasticité » également appelé « module d'Young » est la constante qui relie la contrainte de traction (ou de compression) appliquée à un matériau ($\sigma$) et la déformation (allongement relatif) qui en résulte ($\varepsilon$) pour un matériau élastique isotrope, tant que la déformation reste petite et que la limite d'élasticité du matériau n'est pas atteinte : $\sigma = E\varepsilon$ (loi de Hooke). Le module d'élasticité caractérise la rigidité du matériau.

En règle générale, pour les matériaux métalliques, le module d'élasticité est une grandeur liée à la raideur des liaisons atomiques et il est par conséquent constant pour une composition atomique donnée. C'est ce que l'on désigne ici, dans la présente description, par « propriétés élastiques » ou encore élasticité classique, dite encore enthalpique ou intrinsèque : la contrainte mécanique appliquée au matériau n'induit pas de transformation de phase au sein du matériau.

Les matériaux « superélastiques » sont des matériaux pour lesquels l'application d'une contrainte induit une transformation de phase, via une transformation martensitique réversible. Ainsi, à titre d'exemple, les alliages de titane superélastiques de structure cubique centrée (également désignée structure $\beta$) se transforment sous contrainte, de façon réversible, en phase orthorombique (également désignée structure $\alpha''$) de même composition, à une température égale ou légèrement supérieure à la température de transformation martensitique Ms.

« Module d'élasticité apparent » caractérise la rigidité apparente (c'est la dire la relation entre contrainte et déformation) du matériau superélastique dans le domaine de température et de contrainte dans lequel il subit la transformation de phase martensitique.

La « transformation martensitique » désigne une transformation par laquelle la phase haute température, appelée austénite, change de structure cristalline pour adopter une structure moins symétrique appelée martensite. Dans le cas des matériaux à mémoire de forme et notamment superélastiques, cette transformation est réversible de sorte qu'après élévation de la température ou réversion de la contrainte, la transformation inverse se produit. La température de début de refroidissement ou de transformation martensitique est généralement désignée température Ms, et la température de fin est désignée Mf. Les températures de départ et de fin de la transformation inverse ou austénitique sont généralement désignées As et Af respectivement. Lorsque la température est supérieure à Af, la martensite induite sous contrainte n'est pas stable et revient à la phase austénite dès relâchement de la contrainte. Les températures Ms, Mf, As et Af varient généralement avec la composition chimique du matériau métallique.

Au sens de la présente description, l'expression « zone périphérique externe », ou encore le terme « surface », sont employés ici de façon interchangeable et se réfèrent à une zone du matériau (M) en contact avec l'extérieur et dont l'épaisseur peut varier de quelques microns à plusieurs centaines de micromètres, notamment de 100 $\mu$m à 1 mm. Le matériau (M) comprend une seule et unique zone périphérique externe telle que définie ci-dessus.

Selon une variante préférée de la description, le domaine ($\Delta T$) est un domaine correspondant aux conditions d'utilisation du matériau métallique.

De préférence, ($\Delta T$) comprend ou est légèrement supérieur à la température de transformation martensitique (Ms) de la microstructure ($m_1$) à la pression atmosphérique. De préférence, ($\Delta T$) est compris entre (Ms - 50)°C et (Ms + 100)°C. Ainsi, lors de l'utilisation du matériau (M) comme implant dentaire par exemple, à une température d'utilisation comprise dans le domaine ($\Delta T$), la surface du matériau va subir à la température d'utilisation, une transformation martensitique qui va permettre de réduire le module apparent. De préférence ($\Delta T$) est une température comprise entre 35 et 40°C à la pression atmosphérique.

Selon un aspect préféré, le matériau métallique (M) est biocompatible.

Par « biocompatible », on entend un matériau qui est adapté à une mise en contact avec des tissus humains ou animaux, sans induire de toxicité excessive, d'inflammation, notamment d'irritation, ou de réponse allergique, ou autre effet secondaire indésirable, et qui offre un rapport bénéfice/risque acceptable. Le matériau métallique (M) est un alliage de titane, de préférence de type $\beta$, notamment un alliage dans lequel le titane représente environ 75% ou au moins 70 % (% at.) du matériau métallique (M), en particulier un alliage comprenant en outre du niobium. Les pourcentages indiqués ici, exprimés en % atomique (at. %), se réfèrent à la fraction molaire du titane dans le matériau, *i.e.* au nombre de moles de titane pour cent moles d'atomes du matériau.

A titre d'exemple, on peut citer notamment l'alliage Ti-24Nb. Les alliages de titane sont en effet particulièrement intéressants de par leur résistance à la biocorrosion, leur biocompatibilité et leurs propriétés mécaniques, notamment leur faible module d'élasticité qui peut être relativement proche de celui de l'os. La microstructure ($m_1$) est constituée d'une phase $\beta$(cubique centrée). La microstructure ($m_2$) comprend une phase $\beta$ et une phase $\alpha''$ (orthorombique).

Selon un aspect préféré, l'écart entre les modules d'élasticité $E_2$ et $E_1$ est supérieur à 20 GPa.

Selon une variante préférée, le module d'élas-

ticité apparent $E_1$ est compris entre 20 et 50 GPa, soit une valeur très proche du module d'élasticité de l'os.

Selon une variante préférée, $E_2$ est compris entre 70 et 90 GPa.

De façon avantageuse, le module d'élasticité ($E_1$) est compris entre 10 et 30 GPa et le module d'élasticité ($E_2$) est compris entre 70 et 90 GPa. Ce mode de réalisation est particulièrement intéressant pour la réalisation d'implants dentaires. La surface externe du matériau (M) en contact avec l'os possède un module ($E_1$) très proche de celui de l'os, ce qui permet d'assurer un transfert des contraintes fonctionnelles optimisé et de dissiper une partie de l'énergie mécanique. Le coeur du matériau (M), destiné à former la face interne de ce même implant, en contact avec le pilier, possède un module d'élasticité ($E_2$) élevé permettant d'assurer la rigidité de l'ensemble implant/pilier/prothèse.

*Implant dentaire*

Un deuxième objet de la description concerne un matériau métallique (M) tel que défini ci-dessus pour son utilisation comme implant chez un individu, en particulier pour son utilisation comme implant dentaire.

Selon un autre objet, la description concerne un implant dentaire comprenant ou constitué d'un matériau métallique tel que défini dans la présente description.

La description concerne notamment un implant dentaire comprenant un corps d'implant constitué d'un matériau métallique tel que défini ci-dessus.

*Procédés de préparation du matériau métallique M à gradient d'élasticité*

Selon un autre objet, la description concerne également deux procédés de préparation des matériaux (M) tels que définis ci-dessus. Ces procédés sont chacun basés sur un traitement thermomécanique simple permettant de créer un gradient de microstructure ($m_1$, $m_2$) à l'origine d'un gradient d'élasticité ($E_1$, $E_2$), au sein d'un matériau métallique monolithique M pouvant présenter, pour certaines microstructures, des propriétés superélastiques dans un domaine ($\Delta T$) tel que défini ci-dessus et à pression atmosphérique. Ces deux procédés utilisent notamment le « régime transitoire » lié à tout transfert thermique, et notamment le gradient d'évolution de la microstructure à partir d'un état initial contrôlé : état sévèrement déformé, dans le cas du procédé A dit de « recuit flash », ou état monophasé beta, dans le cas du procédé B dit de « trempe ».

La description concerne également un troisième procédé (C) dit de « dissolution préférentielle ».

*Procédé A - « Recuit flash »*

Selon un aspect, la description concerne un procédé de préparation d'un matériau métallique (M) à gradient d'élasticité tel que défini ci-dessus, comprenant les étapes de :

    i) Déformation à froid d'un matériau métallique monolithique (M), de manière à engendrer un état microstructural ($m_0$) ;

    ii) Traitement thermique dudit matériau obtenu à l'étape (i) à une température comprise entre 500°C et 800°C, pendant une durée suffisamment courte pour obtenir un matériau (M) comprenant les états microstructuraux ($m_1$) et ($m_2$) distincts de ($m_0$) et les modules d'élasticité ($E_1$) et ($E_2$) tels que définis ci-dessus.

Ce procédé conduit à l'obtention d'un gradient de microstructure, créé par la différence de taux de recristallisation entre la surface et le coeur du matériau M. En surface, le traitement conduit à une recristallisation importante donnant une microstructure à grains ($m_1$) qui induit localement une superélasticité réversible et un abaissement du module élastique apparent à une valeur ($E_1$). Au coeur du matériau M, le procédé de recristallisation reste incomplet et permet donc au matériau de rester écroui, empêchant ainsi l'obtention de propriétés superélastiques : le module élastique ($E_2$) est donc localement plus élevé qu'à la surface (module d'élasticité $E_1$). Ce procédé repose donc sur l'utilisation d'un gradient thermique, ici lors de l'étape ii), également appelée « recuit flash », pour créer un gradient de microstructure ($m_1 \neq m_2$) et donc d'élasticité ($E_1 \neq E_2$).

Par « déformation à froid », on entend une déformation réalisée à une température ne permettant pas au matériau de se restaurer. Il conserve donc toutes les marques de la déformation plastique à la fin de l'opération (dislocations, mâcles, phase martensitique induite sous contrainte,...). A l'issue de cette étape, le matériau M se trouve dans un seul et même état microstructural $m_0$, uniforme dans tout l'échantillon. Le matériau M peut être notamment mis en forme sous forme de barreau par tréfilage.

La température choisie pour réaliser le traitement thermique à l'étape ii) est une température permettant d'obtenir la recristallisation de la périphérie externe du matériau M sous une microstructure ($m_1$) possédant un module d'élasticité ($E_1$) et des propriétés superélastiques à la pression atmosphérique et dans un domaine ($\Delta T$). Cette température de recristallisation permet de recristalliser la phase qui sous contrainte se transforme, en formant de petits grains et sans autres précipités. Elle dépend notamment de la composition chimique du matériau monolithique M, tandis que la cinétique de cette étape de recristallisation est fonction également des contraintes thermo-mécaniques subies par le matériau. La température de recristallisation d'un matériau métallique donné peut être aisément déterminée par un homme du métier par des essais de routine, par exemple par mesures de résistivité électrique ou par calorimétrie différentielle. Le matériau métallique (M) est un alliage de titane et niobium. Selon un aspect préféré, la déformation à froid du matériau ($M_0$) est réalisée à l'étape i) à une température comprise entre 15 et 200 °C, en particulier lorsque M est un alliage de titane superélastique. Le traitement thermique à l'étape ii) est réalisé à une température comprise entre 500 et 800 °C, en particulier entre 500 et 700 °C, notamment à 600 °C, en particulier lorsque M est un alliage de titane superélastique.

Selon ce procédé, le traitement thermique est réalisé sur une durée suffisamment courte pour obtenir un matériau M à gradient d'élasticité tel que défini précédemment, *i.e.* un matériau (M) comprenant au moins deux états micro structuraux $m_1$ et $m_2$, ayant respectivement les modules d'élasticité $E_1$ et $E_2$ tels que définis ci-dessus. A titre d'exemple, une durée comprise entre 30 secondes et 6 minutes est généralement suffisante, notamment lorsque le procédé est appliqué à un matériau (M) ayant pour épaisseur maximale 0,5 mm, *i.e* une épaisseur inférieure à 0,5 mm. La durée nécessaire et suffisante à l'obtention du gradient de microstructure et d'élasticité peut être aisément déterminée par l'homme du métier selon des techniques conventionnelles.

Selon une variante, le procédé selon la description comprend en outre une étape subséquente d'usinage du matériau (M) obtenu.

A titre d'exemple le matériau (M) obtenu à l'étape ii) peut être un cylindre ou un lingot qui est ensuite usiné de manière à lui conférer une forme adaptée à l'usage auquel le matériau est destiné, par exemple sous la forme d'un implant dentaire.

Selon un autre mode de réalisation préféré, le procédé comprend en outre une étape d'usinage du matériau ($M_0$) de manière à lui faire acquérir une forme adaptée à l'utilisation souhaitée avant l'étape ii) de traitement thermique, en particulier une forme adaptée pour une utilisation à titre d'implant dentaire.

### Procédé B - « Trempe»

Selon un autre aspect, la description concerne un procédé de préparation d'un matériau métallique (M) à gradient d'élasticité tel que défini ci-dessus, comprenant les étapes de :

> i) Traitement de mise en solution d'un matériau métallique monolithique (M), à une température comprise dans le domaine de stabilité de la phase β, de préférence pendant une durée suffisante pour obtenir un matériau homogène du point de vue de la composition chimique et de la phase cristalline ; et
> ii) Trempe du matériau obtenu à l'étape i) de manière à obtenir un matériau métallique (M) à gradient d'élasticité tel que défini ci-dessus.

Par « traitement de mise en solution », on entend un traitement thermique dans lequel le matériau est maintenu à une température suffisante, et, suffisamment longtemps pour que les précipités non stables disparaissent, que les atomes diffusent et que le matériau soit finalement homogène (phases cristallines en présence et composition chimique). Typiquement, cette température se situe, pour les alliages de titane, à une température de 50°C au-delà du transus beta.

Pour les alliages de titane, la phase pouvant se transformer sous contrainte, ie la phase cubique centrée (ou β) est stable à haute température (environ 850°C). L'étape de traitement de mise en solution i) des alliages de titane peut ainsi être réalisée à une température comprise entre 800°C et 1000°C, de préférence sous un vide secondaire compris entre $10^{-5}$ et $10^{-7}$ Pa, ou bien sous atmosphère contrôlée en présence d'un gaz inerte tel que l'argon ou l'hélium. L'étape de trempe (ii) permet de la conserver à l'identique, à température ambiante si la composition est bien choisie et la vitesse de refroidissement suffisante. La phase obtenue est stable jusqu'à, au moins, 100°C.

Pour obtenir un gradient de microstructure ($m_1$, $m_2$) et d'élasticité ($E_1$, $E_2$) tel que défini dans la présente demande, ce procédé utilise la trempabilité naturelle des matériaux métalliques. En effet, pour un matériau métallique monolithique, la vitesse de refroidissement est notamment fonction de la diffusivité thermique du matériau, et est généralement bien plus importante aux bords qu'au centre. Il est ainsi possible de contrôler le gradient de vitesse de refroidissement en adaptant la sévérité du milieu de trempe (du milieu le plus sévère: l'eau vers le milieu le moins sévère: l'air). De cette manière, il est possible de créer un gradient de microstructure ($m_1$, $m_2$) permettant l'obtention du gradient élastique ($E_1$, $E_2$). Le matériau trempé sévèrement en surface, possède une microstructure monophasée ($m_1$) capable de subir une transformation martensitique sous contrainte, et présentant un faible module élastique ($E_1$). A contrario, selon ce procédé, le coeur du matériau reste imparfaitement trempé, ce qui induit la précipitation de phases annexes qui empêchent cette même transformation martensitique. Ce phénomène est à l'origine du gradient de module élastique recherché.

De préférence, l'étape i) est réalisée à une température comprise entre 800 et 900 °C.

De préférence, l'étape de trempe est réalisé dans un milieu ayant une sévérité suffisamment faible pour permettre l'obtention d'un matériau (M) à gradient d'élasticité tel que défini ci-dessus. La « sévérité » du milieu de trempe est définie ici comme la capacité du milieu à évacuer l'énergie thermique du matériau M.

De façon préférentielle, la sévérité du milieu de trempe est inférieure ou égale à celle de l'eau. Selon un mode de réalisation, la trempe est réalisée dans l'eau.

En effet, si la sévérité du milieu est insuffisante, la vitesse de refroidissement du matériau sera telle que d'autres phases peuvent se tonner à coeur. Or, ces phases peuvent réduire localement la proportion de la phase cubique centrée au sein du matériau et diminuer ainsi le caractère superélastique du matériau M. Lorsqu'elles sont présentes dans une proportion importante, elles peuvent même conduire à la stabilité complète du matériau. Sans superélasticité, le module est alors plus élevé (70-80 GPa, contre 30-40 GPa avec l'effet superélastique pour des alliages de titane).

*Procédé C - « Dissolution préférentielle »*

Selon un autre aspect, la description concerne un procédé de préparation d'un alliage de titane monolithique (M) tel que défini ci-dessus, comprenant les étapes de:

i) Premier traitement thermique d'un alliage de titane (M) à une température où ledit alliage présente une microstructure biphasée ($\alpha$ + $\beta$) ($m_2$), pendant une durée suffisamment longue pour que la microstructure $m_2$ soit homogène sur tout le volume de l'alliage ;

ii) Deuxième traitement thermique de l'alliage de microstructure ($m_2$) obtenu à une température supérieure au transus $\beta$ dudit alliage, pendant une durée suffisamment courte pour obtenir :

- une transformation de la microstructure biphasée ($\alpha$ + $\beta$) ($m_2$) en une microstructure ($m_1$) monophasée $\beta$ sur la zone périphérique externe de l'alliage, et
- une conservation de la microstructure biphasée ($\alpha$ + $\beta$) ($m_2$) au coeur de l'alliage,

ce par quoi on obtient un alliage de titane (M) comprenant les états microstructuraux ($m_1$) et ($m_2$), et les modules d'élasticité ($E_1$, $E_2$), tel que défini ci-dessus.

L'étape i) est réalisée dans le domaine de température où l'alliage présente une microstructure biphasée ($\alpha$ + $\beta$), c'est-à-dire dans le domaine de précipitation de la phase $\alpha$.

La durée pendant laquelle ce traitement est réalisé varie avec le volume du matériau. Plus ce volume est important, plus la durée de ce traitement thermique sera longue.

L'étape ii) est réalisée à une température supérieure au transus $\beta$ de l'alliage. Le transus $\beta$ qui est une température constante pour un alliage de composition chimique donnée, peut être facilement mesuré par des méthodes bien connues de l'homme du métier.

Cette étape est réalisée pendant une durée suffisamment courte pour que la dissolution de la phase $\alpha$ en phase $\beta$ n'ait le temps de se produire que sur la zone périphérique externe du matériau (M), la microstructure biphasée ($\alpha$ + $\beta$), au coeur du matériau (M) restant inchangée.

L'alliage ainsi obtenu comprend une zone périphérique externe constituée d'une microstructure $m_1$ monophasée $\beta$, et un coeur constitué d'une microstructure $m_2$ biphasée ($\alpha$ + $\beta$).

La microstructure biphasée ($\alpha$ + $\beta$) ayant un module d'élasticité $E_2$ très supérieur au module d'élasticité $E_1$ de la microstructure monophasée $\beta$, le gradient de microstructure ($m_1$, $m_2$) produit un gradient d'élasticité ($E_1$, $E_2$) de la périphérie vers le centre de l'alliage.

FIGURES

Figures 1 : représentation d'un implant dentaire (1) mis en place dans l'os maxillaire (2) et surmonté d'une prothèse (4) au niveau de la gencive (3).
Figure 2 : représentation d'un implant dentaire comprenant un corps d'implant (5) destiné à recevoir un pilier (6) permettant de fixer la prothèse.
Figure 3: Spectres de diffraction pour les trois échantillons : tréfilé à froid, recuit flash T1 et recuit flash T2.
Figure 4: Module d'Young mesuré par microindentation instrumentée, le long d'un rayon pour trois échantillons : tréfilé à froid, recuit flash T1 et recuit flash T2.

EXEMPLES

L'alliage de composition Ti-24Nb (% at.) est obtenu par fusion à arc des deux métaux purs. La composition de ce lingot de 20 g est homogénéisée par un traitement thermique à 950°C pendant 20 heures, sous un vide poussé de $10^{-6}$ Pa. L'échantillon est ensuite trempé après un recuit à 850°C de 360 s, dans le but de conserver la phase haute température béta, plus ductile que la phase stable à basse température : alpha. Un tréfilage à température ambiante permet ensuite de passer d'un lingot ayant un diamètre d'environ 11 mm, à une tige de diamètre 4 mm. Une tranche de 2 mm est découpée afin de caractériser le matériau à l'état « tréfilé à froid ». Une autre pièce de 1 cm de long est aussi découpée depuis la tige tréfilée, puis recuite 360 s à 600°C dans un tube de silice rempli d'hélium et plongé dans des bains de sel. A la fin de ce temps, le tube est refroidi rapidement dans de l'eau.

Deux sections transverses du cylindre sont découpées après recuit avec une épaisseur de 2 mm. L'une provient de l'extrémité du cylindre (« recuit flash T1 ») et permettra d'analyser la microstructure et le comportement mécanique à la surface, l'autre est extraite au milieu (« recuit flash T2 ») et permettra de visualiser si le gradient thermique lors du recuit crée un gradient visible pour la microstructure et le comportement mécanique.

L'équipement de la diffraction X est un Philips PW1710 avec un tube de cuivre ($\lambda_{K\alpha1}$=1,542 Å) opérant à 40 kV et 25 mA. Le micro-indenteur instrumenté est un CSM. La pointe utilisée est en diamant et de type Vickers. La force F et le déplacement h de la pointe sont enregistrés durant un cycle dont la force maximale est 3000 mN (atteinte en 30 s). La partie décharge est purement élastique, elle est donc liée directement au module d'Young. Le calcul suit les modèles classiques de Sneddon qui relie la pente en début de décharge et le module d'Young, et d'Oliver et Pharr qui relie la profondeur mesurée h à la profondeur réelle $h_c$ [réf].

L'aire de contact projetée, nécessaire à l'estimation de E, a été calibrée en se basant sur la mesure de silice fondue. Le résultat est le suivant:

$$A_c = 24.5 \times h_c^{\,2} + 1190 \times h_c$$

La fonction d'aire pour une Vickers idéale est 24.5 $h_c^2$, le terme supplémentaire prend en compte les défauts de géométrie. 20 essais sont réalisés tous les 100 $\mu$m le long d'un rayon.

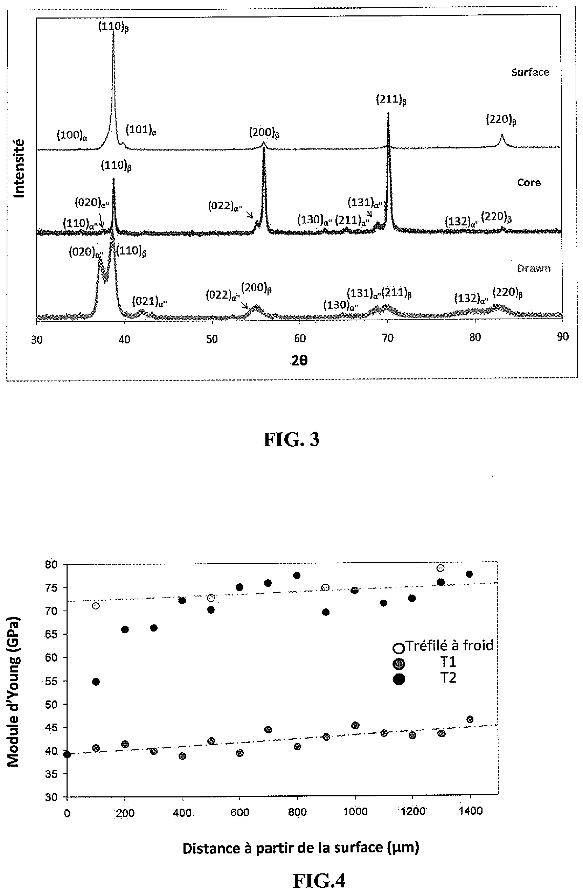

La microstructure des échantillons « tréfilés à froid », « recuit flash T1 » et « recuit flash T2 » est estimée par diffraction des rayons X (Figure 3). L'idée est de comparer les diffractogrammes afin d'estimer l'effet du gradient thermique sur la microstructure. L'échantillon tréfilé à froid a des pics larges et une proportion importante de phase $\alpha$". Ces deux aspects sont la résultante des défauts induits par la déformation plastique : densité de dislocation importante et phase martensitique induite sous contrainte résiduelle. L'échantillon « recuit flash T2 » est texturé puisque le pic principal $\beta$ est (211) et non (110). On peut aussi identifier les phases $\beta$ et $\alpha$". Le résultat est relativement proche du « tréfilé à froid » mais deux différences significatives peuvent être indiquées : les pics sont plus étroits et la proportion de phase $\alpha$" est réduite. L'échantillon « recuit flash T1 » est composé de la phase $\beta$ principalement et de quelques pourcents de $\alpha$. Cela signifie que la phase $\alpha$" disparait et que la phase $\beta$ recristallise. L'énergie est même suffisante pour former quelques cristaux de $\alpha$.

Ces résultats représentent une microstructure moyenne des différentes sections transverses: « tréfilé à froid », « recuit flash T1 » et « recuit flash T2 ». Les deux premiers sont homogènes a priori mais le troisième est apparemment hétérogène. En effet, la zone périphérique pour T2 est identique à T1 puisqu'il s'agit d'une surface. Or, le spectre de diffraction de T2 montre principalement une phase $\beta$ texturée et la présence de $\alpha$". La conclusion est, par conséquent, que l'échantillon T2 présente un gradient de microstructure et que la proportion de $\alpha$" est probablement plus grande au centre de l'échantillon que ce qu'on peut estimer par diffraction des rayons X.

Le comportement mécanique a ensuite été étudié localement par microindentation instrumentée. La figure 4 présente les valeurs de module d'Young pour les trois échantillons. E est constant le long du rayon pour le « tréfilé à froid » (75 GPa) et le « recuit flash T1 » (40 GPa). Pour le recuit flash T2, le module croit progressivement de 40 GPa à 75 GPa sur une zone de 400 $\mu$m environ, et reste ensuite constant. Cela démontre que le gradient de microstructure peut avoir un effet sur le comportement élastique, pour cette composition et dans ces conditions d'élaboration.

## Revendications

1. Alliage de titane monolithique (M) pour son utilisation comme implant chez un individu, dans lequel le titane représente au moins 70 % en pourcentage atomique de l'alliage de titane (M), ledit alliage comprenant en outre du niobium,
   ledit alliage comprenant, dans un domaine de température ($\Delta$T) comprise entre 35 et 40°C et à pression atmosphérique :

   - une seule et unique zone périphérique externe constituée d'une microstructure ($m_1$) monophasée $\beta$ ayant un module d'élasticité ($E_1$) et possédant des propriétés superélastiques dans ledit domaine ($\Delta$T), et
   - un coeur constitué d'une microstructure ($m_2$) biphasée $\alpha + \beta$ ou comprenant une phase $\beta$ et une phase $\alpha$", ladite microstructure ($m_2$) ayant un module d'élasticité ($E_2$), et possédant des propriétés élastiques dans ledit domaine ($\Delta$T) ;
   lesdites microstructures ($m_1$) et ($m_2$) étant distinctes l'une de l'autre, et
   ledit module d'élasticité ($E_1$) étant inférieur audit module d'élasticité ($E_2$).

2. Alliage de titane (M) pour son utilisation selon la revendication 1, dans lequel l'écart entre $E_1$ et $E_3$ est supérieur ou égal à 20 GPa.

3. Alliage de titane (M) pour son utilisation selon l'une quelconque des revendications précédentes, dans lequel $E_1$ est compris entre 20 et 50 GPa.

4. Alliage de titane (M) pour son utilisation selon l'une quelconque des revendications précédentes, dans lequel $E_2$ est compris entre 70 et 90 GPa.

5. Alliage de titane (M) selon la revendication 4, pour son utilisation comme implant dentaire.

6. Implant dentaire comprenant ou constitué d'un alliage de titane (M) tel que défini dans les revendications 1 à 5.

7. Implant dentaire selon la revendication 6, comprenant un corps d'implant constitué d'un matériau métallique (M).

8. Procédé de préparation d'un alliage de titane (M) selon l'une quelconque des revendications 1 à 5, comprenant les étapes de :

   i) Déformation à froid d'un alliage de titane monolithique (M), de manière à engendrer un état microstructural ($m_0$) ;
   ii) Traitement thermique dudit alliage obtenu à l'étape (i) à une température comprise entre 500°C et 800°C, pendant une durée suffisamment courte de manière à obtenir un alliage de titane (M) comprenant les états microstructuraux ($m_1$) et ($m_2$) distincts de ($m_0$), et les modules d'élasticité ($E_1$, $E_2$), tels que définis aux revendications 1 à 5.

9. Procédé selon la revendication 8, dans lequel la déformation à froid du matériau (M) est réalisée à une température comprise entre 15 et 200°C.

10. Procédé selon l'une quelconque des revendications précédentes 8 ou 9, dans lequel le traitement thermique est réalisé à une température comprise entre 500 et 700°C.

11. Procédé selon l'une quelconque des revendications précédentes 9 à 11, dans lequel le traitement thermique est réalisé sur une durée comprise entre 30 secondes et 6 minutes.

12. Procédé selon l'une quelconque des revendications précédentes 8 à 11, comprenant en outre une étape subséquente à l'étape ii) d'usinage de l'alliage (M) obtenu.

13. Procédé selon l'une quelconque des revendications précédentes 8 à 11, comprenant en outre une étape de mise en forme de l'alliage (M) de manière à lui faire acquérir une forme adaptée à l'utilisation souhaitée avant l'étape ii) de traitement thermique.

14. Procédé selon la revendication 13, dans lequel la forme de l'alliage (M) est adaptée pour une utilisation à titre d'implant dentaire.

15. Procédé de préparation d'un alliage de titane (M) tel que défini dans l'une quelconque des revendications 1 à 5, comprenant les étapes de :

    i) Traitement de mise en solution d'un alliage de titane monolithique (M), à une température comprise dans le domaine de stabilité de la phase $\beta$, de préférence pendant une durée suffisante pour obtenir un matériau homogène du point de vue de la composition chimique et de la phase cristalline ; et
    ii) Trempe du matériau obtenu à l'étape i) dans de l'eau, de manière à obtenir un alliage de titane (M) comprenant les états microstructuraux ($m_1$) et ($m_2$), et les modules d'élasticité ($E_1$, $E_2$), tel que défini aux revendications 1 à 5.

16. Procédé selon la revendication 15, dans lequel l'étape i) est réalisée à une température comprise entre 800 et 900°C.

17. Procédé de préparation d'un alliage de titane monolithique (M) tel que défini dans l'une quelconque des revendications 1 à 5, comprenant les étapes de :

    i) Premier traitement thermique d'un alliage de titane (M) à une température où ledit alliage présente une microstructure biphasée ($\alpha + \beta$) ($m_2$), pendant une durée suffisamment longue pour que la microstructure $m_2$ soit homogène sur tout le volume de l'alliage ;
    ii) Deuxième traitement thermique de l'alliage de microstructure ($m_2$) obtenu à une température supérieure au transus $\beta$ dudit alliage, pendant une durée suffisamment courte pour obtenir :

        - une transformation de la microstructure biphasée ($\alpha + \beta$) ($m_2$) en une microstructure ($m_1$) monophasée $\beta$ sur la zone périphérique externe de l'alliage, et
        - une conservation de la microstructure biphasée ($\alpha + \beta$) ($m_2$) au coeur de l'alliage,

ce par quoi on obtient un alliage de titane (M) comprenant les états microstructuraux ($m_1$) et ($m_2$), et les modules d'élasticité ($E_1$, $E_2$), tel que défini ci-dessus.

**Patentansprüche**

1. Monolithische Titanlegierung (M) zur Verwendung als Implantat bei einem Individuum, wobei Titan mindestens 70 Atom-% der Titanlegierung (M) ausmacht, wobei die Legierung weiter Niob umfasst, wobei die Legierung in einem Temperaturbereich ($\Delta T$) zwischen 35 und 40 °C und bei Atmosphärendruck umfasst:

    - einen einzigen äußeren Randbereich, der aus einer einphasigen, $\beta$, Mikrostruktur ($m_1$) besteht, die einen Elastizitätsmodul ($E_1$) aufweist und in dem Bereich ($\Delta T$) superelastische Eigenschaften besitzt, und
    - einen Kern, der aus einer zweiphasigen, $\alpha + \beta$, Mikrostruktur ($m_2$) besteht oder eine Phase $\beta$ und eine Phase $\alpha''$ umfasst, wobei die Mikrostruktur ($m_2$) einen Elastizitätsmodul ($E_2$) aufweist und in dem Bereich ($\Delta T$) elastische Eigenschaften besitzt;

wobei die Mikrostrukturen ($m_1$) und ($m_2$) voneinander verschieden sind, und
wobei der Elastizitätsmodul ($E_1$) kleiner ist als der Elastizitätsmodul ($E_2$).

2. Titanlegierung (M) zur Verwendung nach Anspruch 1, wobei die Differenz zwischen $E_1$ und $E_2$ größer oder gleich 20 GPa ist.

**3.** Titanlegierung (M) zur Verwendung nach einem der vorstehenden Ansprüche, wobei $E_1$ zwischen 20 und 50 GPa liegt.

**4.** Titanlegierung (M) zur Verwendung nach einem der vorstehenden Ansprüche, wobei $E_2$ zwischen 70 und 90 GPa liegt.

**5.** Titanlegierung (M) nach Anspruch 4, zur Verwendung als Zahnimplantat.

**6.** Zahnimplantat, umfassend oder bestehend aus eine/r Titanlegierung (M), wie in den Ansprüchen 1 bis 5 definiert.

**7.** Zahnimplantat nach Anspruch 6, umfassend einen Implantatkörper, der aus einem metallischen Material (M) besteht.

**8.** Verfahren zur Herstellung einer Titanlegierung (M) nach einem der Ansprüche 1 bis 5, umfassend die Schritte des:

   i) Kaltverformens einer monolithischen Titanlegierung (M), um einen mikrostrukturellen Zustand ($m_0$) zu bewirken;
   ii) Wärmebehandelns der im Schritt (i) erhaltenen Legierung bei einer Temperatur zwischen 500 °C und 800 °C während einer ausreichend kurzen Dauer, um eine Titanlegierung (M) zu erhalten, die die mikrostrukturellen Zustände ($m_1$) und ($m_2$), die von ($m_0$) verschieden sind, und die Elastizitätsmodule ($E_1$, $E_2$), wie in den Ansprüchen 1 bis 5 definiert, umfasst.

**9.** Verfahren nach Anspruch 8, wobei das Kaltverformen des Materials (M) bei einer Temperatur zwischen 15 und 200 °C ausgeführt wird.

**10.** Verfahren nach einem der vorstehenden Ansprüche 8 oder 9, wobei die Wärmebehandlung bei einer Temperatur zwischen 500 und 700 °C ausgeführt wird.

**11.** Verfahren nach einem der vorstehenden Ansprüche 9 bis 11, wobei die Wärmebehandlung über eine Dauer zwischen 30 Sekunden und 6 Minuten ausgeführt wird.

**12.** Verfahren nach einem der vorstehenden Ansprüche 8 bis 11, weiter nach dem Schritt ii) einen Schritt des Bearbeitens der erhaltenen Legierung (M) umfassend.

**13.** Verfahren nach einem der vorstehenden Ansprüche 8 bis 11, weiter einen Schritt des Formgebens der Legierung (M) umfassend, um ihr vor dem Schritt ii) der Wärmebehandlung eine für die gewünschte Verwendung geeignete Form zu verleihen.

**14.** Verfahren nach Anspruch 13, wobei die Form der Legierung (M) für eine Verwendung als Zahnimplantat geeignet ist.

**15.** Verfahren zur Herstellung einer Titanlegierung (M) wie in einem der Ansprüche 1 bis 5 definiert, umfassend die Schritte des:

   i) Lösungsglühens einer monolithischen Titanlegierung (M) bei einer Temperatur im Stabilitätsbereich der Phase β bevorzugt während einer Dauer, die ausreicht, um ein Material zu erhalten, das in Hinblick auf die chemische Zusammensetzung und die kristalline Phase homogen ist; und
   ii) Abschreckens des im Schritt i) erhaltenen Materials in Wasser, um eine Titanlegierung (M) zu erhalten, die die mikrostrukturellen Zustände ($m_1$) und ($m_2$) und die Elastizitätsmodule ($E_1$, $E_2$), wie in den Ansprüchen 1 bis 5 definiert, umfasst.

**16.** Verfahren nach Anspruch 15, wobei der Schritt i) bei einer Temperatur zwischen 800 und 900 °C ausgeführt wird.

**17.** Verfahren zur Herstellung einer monolithischen Titanlegierung (M) wie in einem der Ansprüche 1 bis 5 definiert, umfassend die Schritte der:

   i) ersten Wärmebehandlung einer Titanlegierung (M) bei einer Temperatur, bei der die Legierung eine zweiphasige (α + β) Mikrostruktur ($m_2$) aufweist, während einer ausreichend langen Dauer, damit die Mikrostruktur $m_2$ über das gesamte Volumen der Legierung homogen ist;
   ii) zweiten Wärmebehandlung der erhaltenen Mikrostrukturlegierung ($m_2$) bei einer Temperatur oberhalb des β-Transus der Legierung während einer ausreichend kurzen Dauer, um zu erhalten:

      - eine Umwandlung der zweiphasigen (α + β) Mikrostruktur ($m_2$) in eine einphasige, β, Mikrostruktur ($m_1$) am äußeren Randbereich der Legierung,
      - eine Bewahrung der zweiphasigen (α + β) Mikrostruktur ($m_2$) im Kern der Legierung,

wodurch eine Titanlegierung (M) erhalten wird, die die mikrostrukturellen Zustände ($m_1$) und ($m_2$) und die Elastizitätsmodule ($E_1$, $E_2$), wie obenstehend definiert, umfasst.

## Claims

1. Monolithic titanium alloy (M) for use as an implant in an individual, in which titanium represents at least 70% by atomic percentage of the titanium alloy (M), said alloy also comprising niobium, said alloy comprising the following, within a temperature range ($\Delta T$) between 35 and 40°C and at atmospheric pressure:

   - a single and unique external peripheral zone composed of a single phase $\beta$ microstructure ($m_1$) with a modulus of elasticity ($E_1$) and with superelastic properties in said domain ($\Delta T$), and
   - a core composed of a two-phase $\alpha + \beta$ microstructure ($m_2$) or comprising a phase $\beta$ and a phase $\alpha''$, said microstructure ($m_2$) having a modulus of elasticity ($E_2$) and having elastic properties in said domain ($\Delta T$);

   said microstructures ($m_1$) and ($m_2$) being distinct from each other, and
   said modulus of elasticity ($E_1$) being less than said modulus of elasticity ($E_2$).

2. Titanium alloy (M) for use according to claim 1, in which the difference between $E_1$ and $E_2$ is greater than or equal to 20 GPa.

3. Titanium alloy (M) for use according to any one of the previous claims, in which $E_1$ is between 20 GPa and 50 GPa.

4. Titanium alloy (M) for use according to any one of the previous claims, in which $E_2$ is between 70 GPa and 90 GPa.

5. Titanium alloy (M) according to claim 4, for use as a dental implant.

6. Dental implant comprising or composed of a titanium alloy (M) as defined in claims 1 to 5.

7. Dental implant according to claim 6, comprising an implant body composed of a metallic material (M).

8. Method for preparation of a titanium alloy (M) according to any one of claims 1 to 5, including the following steps:

   i) Cold deformation of a monolithic titanium alloy (M), so as to create a microstructural state ($m_0$);
   ii) Heat treatment of said alloy obtained in step (i) at a temperature of between 500°C and 800°C, for a sufficiency short duration so as to obtain a titanium alloy (M) comprising microstructural states ($m_1$) and ($m_2$) distinct from ($m_0$), and moduli of elasticity ($E_1$, $E_2$) as defined in claims 1 to 5.

9. Method according to claim 8, in which the cold deformation of the material (M) is made at a temperature between 15 and 200°C.

10. Method according to any one of the previous claims 8 or 9, in which the heat treatment is carried out at a temperature between 500 and 700°C.

11. Method according to any one of the previous claims 9 to 11, in which the heat treatment is done for a duration of between 30 seconds and 6 minutes,

12. Method according to any one of the previous claims 8 to 11, also including a step subsequent to step ii) for machining of the alloy (M) obtained.

13. Method according to any one of the previous claims 8 to 11, also including a step in which the alloy (M) is formed such that it acquires a shape adapted to the required use before the heat treatment step ii).

14. Method according to claim 13, in which the alloy (M) is adapted for use as a dental implant.

15. Method for preparation of a titanium alloy (M) as defined in any one of claims 1 to 5, including the following steps:

   i) Treatment to create a solution of a monolithic titanium alloy (M) at a temperature within the stability range of phase $\beta$, preferably for a sufficiently long duration to obtain a material with a homogeneous chemical composition and crystalline phase, and
   ii) Quench the material obtained in step i) in water, so as to obtain a titanium alloy (M) comprising microstructural states ($m_1$) and ($m_2$), and moduli of elasticity ($E_1$, $E_2$) as defined in claims 1 to 5.

16. Method according to claim 15, in which step i) is done at a temperature between 800 and 900°C.

17. Method for preparation of a monolithic titanium alloy (M) as defined in any one of claims 1 to 5, including the following steps

   i) First heat treatment of a titanium alloy (M) at a temperature at which said alloy has a two-phase ($\alpha + \beta$) microstructure ($m_2$), for a sufficiency long duration so that the microstructure $m_2$ is homogeneous over the entire volume of the alloy;
   ii) Second heat treatment of the alloy with microstructure ($m_2$) obtained at a temperature higher than the $\beta$ transus of said alloy, for a sufficiently short duration to obtain:

- a transformation of the two-phase $(\alpha + \beta)$ microstructure $(m_2)$ into a single phase $\beta$ microstructure $(m_1)$ over the external peripheral zone of the alloy, and
- conservation of the two-phase $(\alpha + \beta)$ microstructure $(m_2)$ microstructure in the core of the alloy,

as a result of which a titanium alloy (M) comprising microstructural states $(m_1)$ and $(m_2)$ is obtained, and moduli of elasticity $(E_1, E_2)$ as defined above.

**FIG. 1**

**FIG. 2**

**FIG. 3**

**FIG.4**